# EUROPÄISCHE PATENTANMELDUNG

(11) **EP 2 165 669 A1**
(43) Veröffentlichungstag der Anmeldung: **24.03.2010**
(21) Anmeldenummer: 09012069.2
(22) Anmeldetag: 23.09.2009
(51) Int. Cl.: A61B 18/20

(54) **Verfahren und Vorrichtung zur kosmetischen Hautbehandlung**

(30) Priorität: 23.09.2008 DE 102008048409
(71) Anmelder: megasun Invest AG, 9422 Staad (CH)
(72) Erfinder: Markus Hahl, 83607 Holzkirchen/Kleinhartpenning (DE)
(74) Vertreter: Hauck Patent- und Rechtsanwälte

(57) **Zusammenfassung**

Es werden ein Verfahren und eine Vorrichtung zur kosmetischen Hautbehandlung, insbesondere kosmetischen Haarentfernung, beschrieben. Bei dem Verfahren wird eine Messbestrahlung vor einer Behandlungsbestrahlung durch Beaufschlagen der zu behandelnden, insbesondere zu enthaarenden, Hautstelle mit Pulslicht durchgeführt, oder die zu behandelnde, insbesondere zu enthaarende, Hautstelle wird vor der Behandlungsbestrahlung mit einer Kamera aufgenommen, um ein Hautbild zu erstellen. Die remittierte Messstrahlung wird empfangen und zur Bestimmung von kennzeichnenden Hautparametern ausgewertet, oder das erstellte Hautbild wird zur Bestimmung von kennzeichnenden Hautparametern ausgewertet. In Abhängigkeit von diesen Hautparametern werden Parameter der Behandlungsbestrahlung eingestellt, und die Behandlungsbestrahlung wird mit den eingestellten Behandlungsbestrahlungsparametern durch Beaufschlagen der zu behandelnden, insbesondere zu enthaarenden, Hautstelle mit Pulslicht durchgeführt. Durch die hierdurch erreichte Einstellung der Bestrahlung auf den individuellen Haut/Haartyp lassen sich Schädigungen der betroffenen Hautbereiche weitgehend vermeiden.

## Beschreibung

Die vorliegende Erfindung betrifft ein Verfahren zur kosmetischen Hautbehandlung, insbesondere zur kosmetischen Haarentfernung. Sie ist ferner auf eine Vorrichtung zur Durchführung eines derartigen Verfahrens gerichtet.

Die auf elektromagnetischer Strahlung basierenden Haarentfernungs- und Haarreduktionssysteme (optische Verfahren) basieren auf dem Funktionsprinzip, dass elektromagnetische Strahlung auf die Haut appliziert und vom Haar (der Haarwurzel) bzw. dem innerhalb des Haares befindlichen Farbstoff (Pigment/Melanin) absorbiert wird. Infolge einer Überbelastung - es wirkt mehr Strahlung auf das Pigment als vom Pigment absorbiert werden kann - und nicht zuletzt durch die Absorption selbst entsteht thermische Energie. Die erzeugte thermische Energie führt zu einer inneren Umwandlung innerhalb der Melanosomen. Sobald die Wärmeenergie an der Haarpapille 70 °C überschreitet, führt dies zur Degeneration des Haares. Diese Degeneration kann sowohl die Papille selbst betreffen als auch die entsprechenden Kapillargefäße.

Infolge der vorstehend beschriebenen thermischen Belastung (elektromagnetische Strahlung/innere Umwandlung) kommt es zu einem Verschluss bzw. einer Verödung der Fugen zwischen den Endothelzellen, d.h. einem Verschluss der Fibrin-Netzstruktur. Ab diesem Zeitpunkt ist keine Sauerstoff- und Nährstoffzufuhr zur Papille mehr möglich, was zu einer Degeneration der Papille und damit zu einer dauerhaften Entfernung des Haares führt.

Derartige kosmetische Haarentfernungsverfahren sind bekannt. Hierbei werden beispielsweise hohe elektromagnetische Impulse in Wellenlängenbereichen von 400 nm bis 1.200 nm auf die Haut appliziert. Diese Verfahren werden jedoch aus dermatologischer Sicht kritisch betrachtet. Problematisch ist bei derartigen Verfahren, dass die Energie des Spektrums nicht nur vom Melanin, wie gewünscht, sondern auch vom umgebenden Hautbereich absorbiert wird. Um diesen Effekt zu minimieren, wird häufig mit geringer Intensität gearbeitet. Das hat jedoch den Nachteil, dass dann eine ausreichende Schädigung der Follikel nicht mehr gegeben ist. Um eine entsprechend erfolgreiche Behandlung zu erzielen, werden folglich Behandlungsdosen gewählt, welche eine erhöhte Zellschädigung hervorrufen.

Diese Problematik ist im Wesentlichen darauf zurückzuführen, dass die menschliche Haut von Fall zu Fall einen sehr unterschiedlichen Aufbau aufweist, so dass bei Aufbringung einer identischen elektromagnetischen Strahlung sich unterschiedliche Resultate ergeben. Eine Ursache hierfür sind unterschiedlich dicke Hautschichten. Beispielsweise kann die oberste Hautbarriere, die Hornschicht, durch Umwelteinflüsse bedingt, beispielsweise Sonnenexposition, Hautpflege, Reinigung, mechanische Beanspruchung, eine unterschiedliche Struktur oder Stärke besitzen. Insbesondere spielt hierbei eine Verdickung der Hornschicht, beispielsweise durch eine Besonnung, eine große Rolle, die die Behandlung mittels elektromagnetischer Strahlung beeinflusst. Bedingt durch die streuende Eigenschaft der Hornschicht wird die Strahlung während einer Behandlung verstärkt refraktiert und je nach Spektrum innerhalb der Hornschicht auch absorbiert. Dies kann eine entsprechend reduzierte punktuelle Strahlungsbelastung und somit einen entsprechend reduzierten Behandlungserfolg bedingen.

Ein weiterer Einflussfaktor in Bezug auf eine Haarentfernung ist die eigentliche lokale Position des Haares innerhalb der Haut. Auch die diesbezüglich vorhandenen Unterschiede können bei einer entsprechenden gleichartigen Strahlungsbehandlung zu unterschiedlichen Ergebnissen führen. Auch die Anzahl der pro Epikelzelle vorhandenen Melanosomen sowie die Melanosomart beeinflussen die Haarentfernung durch elektromagnetische Strahlung.

Insgesamt können sich daher die unterschiedlichsten Hauttypen und Haartypen auf eine derartige Strahlungsbehandlung zur Entfernung von Haar auswirken, so dass die Gefahr besteht, dass die durchgeführte Bestrahlungsbehandlung entweder nicht ausreichend wirksam sein oder sich schädigend auf die umgebehden Hautbereiche auswirken kann. Erfindungsgemäß sollen diese Probleme beseitigt werden.

Der vorliegenden Erfindung liegt die Aufgabe zugrunde, ein Verfahren zur kosmetischen Hautbehandlung, insbesondere kosmetischen Haarentfernung, zu schaffen, das sich bei den unterschiedlichsten Haut/Haartypen unter Vermeidung von Schädigungen der betroffenen Hautbereiche besonders effektiv einsetzen lässt.

Diese Aufgabe wird erfindungsgemäß durch ein Verfahren zur Haarentfernung gelöst, das die folgenden Schritte aufweist:
a. Durchführen einer Messbestrahlung vor einer Behandlungsbestrahlung durch Beaufschlagen der zu behandelnden, insbesondere zu enthaarenden, Hautstelle mit Pulslicht oder Aufnehmen der zu behandelnden, insbesondere zu enthaarenden, Hautstelle mit einer Kamera vor einer Behandlungsbestrahlung;
b. Empfangen und Auswerten der remittierten Messstrahlung oder Erstellen eines Hautbildes mit der Kamera und Auswerten desselben zur Bestimmung von den akuten Hautzustand der zu behandelnden, insbesondere zu enthaarenden, Hautstelle kennzeichnenden Hautparametern;
c. Einstellen von Parametern der Behandlungsbestrahlung in Abhängigkeit von den bestimmten Hautparametern; und
d. Durchführen der Behandlungsbestrahlung mit den eingestellten Behandlungsbestrahlungsparametern durch Beaufschlagen der zu behandelnden, insbesondere zu enthaarenden, Hautstelle mit Pulslicht.

Das erfindungsgemäße Verfahren setzt sich daher im Wesentlichen aus zwei Verfahrensschritten zusammen. In einem ersten Schritt werden mit Hilfe einer aufgebrachten Messbestrahlung bestimmte Hautparameter ermittelt, und in einem zweiten Schritt werden Parameter einer nachfolgenden Behandlungsbestrahlung in Abhängigkeit von den bestimmten Hautparametern eingestellt und wird die Behandlungsbestrahlung mit diesen eingestellten Behandlungsstrahlungsparametern aufgebracht. Hierdurch erfolgt eine Anpassung der durchgeführten Behandlungsbestrahlung an den akuten Hautzustand der zu behandelnden, insbesondere zu enthaarenden, Hautstelle, so dass sich eine in Bezug auf die jeweilige Hautstelle optimierte Behandlungsbestrahlung durchführen lässt. Die Gefahr, dass es zu einer wirkungslosen oder zu einer die Hautstelle schädigenden Bestrahlung kommt, kann daher ausgeschaltet werden.

Das erfindungsgemäße Verfahren dient insbesondere zur kosmetischen Haarentfernung. Es sind hiermit jedoch auch andere kosmetische Hautbehandlungen durchführbar, beispielsweise die Entfernung von Hautunreinheiten, die Reduzierung von Falten, Gewebestraffung, Hautverjüngung, Couperose/Besenreiser, die Behandlung von Altershaut, Pigmentflecken etc. Beispielsweise werden bei der Entfernung von Hautunreinheiten die entsprechenden Unreinheiten mit der Messbestrahlung beaufschlagt, und die remittierte Messstrahlung wird empfangen und ausgewertet. Es werden dann Parameter der Behandlungsbestrahlung in Abhängigkeit von den bestimmten Hautparametern, die aus der remittierten Messstrahlung bestimmt wurden, eingestellt, und es wird die Behandlungsbestrahlung der Hautunreinheit durchgeführt.

Zur Durchführung der Messbestrahlung kann die gleiche Strahlungsquelle Verwendung finden, die zur Durchführung der Behandlungsbestrahlung eingesetzt wird. Es können jedoch auch zur Durchführung der Messbestrahlung und zur Durchführung der eigentlichen Behandlungsbestrahlung unterschiedliche Strahlungsquellen verwendet werden. Die Verwendung der gleichen Strahlungsquelle wird jedoch bevorzugt, um das Verfahren weniger aufwendig zu gestalten.

Anstatt der Durchführung einer Messbestrahlung kann auch die zu behandelnde, insbesondere zu enthaarende, Hautstelle vor einer Behandlungsbestrahlung mit einer Kamera aufgenommen werden. Mit dieser Kamera kann dann ein entsprechendes Hautbild erstellt werden, das ausgewertet wird, um den akuten Hautzustand der zu behandelnden, insbesondere zu enthaarenden, Hautstelle kennzeichnende Hautparameter zu ermitteln. Das von der Hautstelle gewonnene optische Bild wird dabei vorzugsweise in einzelne Bildpunkte zerlegt, die in Bezug auf Hell-Dunkel-Werte und/oder Farbwerte ausgewertet werden. Durch Vergleich dieser Werte mit gespeicherten Standardwerten können die entsprechenden Hautparameter ermittelt werden, die zum Einstellen der Parameter der Behandlungsbestrahlung benutzt werden.

Das erfindungsgemäße Verfahren wird vorzugsweise kontinuierlich durchgeführt. So werden vorzugsweise abwechselnd eine Messbestrahlung oder Hautbilderstellung und eine nachfolgende Behandlungsbestrahlung durchgeführt. Insbesondere wird dabei so vorgegangen, dass von der Durchführung einer Messbestrahlung oder Hautbilderstellung und einer nachfolgenden Behandlungsbestrahlung einer zu behandelnden, insbesondere zu enthaarenden, Hautstelle kontinuierlich auf die Durchführung einer Messbestrahlung oder Hautbilderstellung und einer nachfolgenden Behandlungsbestrahlung einer benachbarten zu behandelnden, insbesondere zu enthaarenden, Hautstelle übergegangen wird. Auf diese Weise lässt sich beispielsweise ein zu enthaarender Hautbereich kontinuierlich enthaaren, wobei benachbarte Einzelbereiche sukzessive je einer Messbestrahlung oder Hautbilderstellung und einer nachfolgenden Behandlungsbestrahlung unterzogen werden. Mit anderen Worten, eine erste Hautstelle wird einer Messbestrahlung oder Hautbilderstellung und einer nachfolgenden Behandlungsbestrahlung unterzogen, wonach auf eine benachbarte Hautstelle übergegangen wird, die einer Messbestrahlung oder Hautbilderstellung und einer nachfolgenden Behandlungsbestrahlung unterzogen wird, usw. Diese Vorgehensweise kann fließend, d.h. ohne zwischenzeitliche Stoppphasen, durchgeführt werden.

Bei einer weiteren Variante des erfindungsgemäßen Verfahrens wird vor der Behandlung eines Hautareals mit mehreren Behandlungsbestrahlungsvorgängen eine Messbestrahlung durchgeführt. Hierbei erfolgt daher die Messung nicht vor jedem einzelnen "Schuss", sondern vor jeder Behandlung bezogen auf das jeweilige Hautareal, beispielsweise Gesicht.

Was das erfindungsgemäß eingesetzte Pulslicht betrifft, so wird vorzugsweise mit Pulslicht einer Wellenlänge von 400-1.200 nm, insbesondere 620-1.200 nm, einer Puls- und Pausenlänge von je 2-50 msec und einer Pulsintensität von 1-70 J/cm² gearbeitet. Die entsprechenden, individuell an den gemessenen Hauttyp angepassten Parameter der für die Behandlungsstrahlung eingesetzten Strahlungsquelle werden aus der remittierten Messstrahlung der Messbestrahlung oder dem erstellten Hautbild gewonnen, und zwar werden insbesondere die Absorptions-, Refraktions- und/oder Reflexionsparameter der remittierten Messstrahlung oder die Hell-Dunkel-Werte bzw. Farbwerte der Bildpunkte ermittelt, woraus die den akuten Hautzustand kennzeichnenden Hautparameter bestimmt werden. Als die den akuten Hautzustand kennzeichnenden Parameter werden insbesondere die Dicke der Hautschichten und/oder der Melanosomtyp bestimmt, und in Abhängigkeit von diesen ermittelten Hautparametern werden als Parameter der Behandlungsbestrahlung insbesondere die Wellenlänge, die Puls/Pausenlänge und/oder die Pulsintensität eingestellt. Es kann beispielsweise hierbei mit für einen bestimmten Hauttyp voreingestellten Parametern gearbeitet werden, die in Abhängigkeit von den Auswertungsergebnissen der jeweiligen Messbestrahlung variiert und für den jeweils ermittelten Hauttyp optimal eingestellt werden.

Des Weiteren kann bei dem erfindungsgemäßen Verfahren durch Temperaturmessung der Haarpapille ein die Vorbelastung der Haut mit Strahlung kennzeichnender Parameter bestimmt werden, bei dem es sich ebenfalls um einen den akuten Hautzustand kennzeichnenden Parameter handelt. Auch in Abhängigkeit von diesem Parameter können dann die entsprechenden Parameter der Behandlungsbestrahlung eingestellt werden. Vor jedem Lichtimpuls kann dabei zusätzlich eine Temperaturmessung erfolgen, die eine vorherrschende, höhere Temperatur innerhalb der Zellstrukturen bestimmt. Hierdurch kann ein weiterer Impuls in der Intensität und/oder Dauer reduziert oder das Spektrum angepasst oder der Puls gänzlich unterbunden werden.

Als Messbestrahlung kann vorzugsweise eine leistungsreduzierte Behandlungsbestrahlung eingesetzt werden. Hierbei können entsprechend optisch ausgerichtete Empfänger oder zusätzliche Filter Verwendung finden, die andere Spektralbereiche (< 400 nm und > 460 nm) entsprechend sperren. Grundlage bei dem erfindungsgemäßen Verfahren ist somit eine exakte Klassifizierung der bei der Behandlung zu durchdringenden Schichttiefen der Haut. Vor jeder einzelnen Strahlungsapplikation erfolgt eine Messung bzw. Bilderstellung, gefolgt von einer Einstellung der jeweiligen Behandlungsbestrahlung. Die Behandlungsbestrahlung kann aufgrund der vorherigen Hautmessung in Intensität, spektrale Eigenschaften (Verteilung), Bestrahlungsdauer, Pulsdauer und Pause zwischen den einzelnen Pulse/der Bestrahlung eingestellt werden.

Bei einer weiteren Ausführungsform des erfindungsgemäßen Verfahrens wird eine Behandlungsbestrahlung mit einem geänderten Anwendungsspektrum, insbesondere einer Anreicherung der grünen und blauen Spektralbande, eingesetzt. Dies kann beispielsweise durch eine zusätzliche Strahlungsquelle im grünen und blauen Strahlungsbereich oder aber durch die Reduktion der anderen Spektralbereiche, gefolgt von häufigeren Pulsintervallen, erfolgen.

Eine weitere Ausführungsform des erfindungsgemäßen Verfahrens ist **dadurch gekennzeichnet, dass** vor der Durchführung der Messbestrahlung und/oder Behandlungsbestrahlung die zu behandelnde Hautstelle mit Ultraschall beaufschlagt wird. Durch die Beaufschlagung mit Ultraschall findet ein "Vorheizvorgang" der entsprechenden Hautareale bzw. Hautschichten statt, wobei eine Penetration in tiefere Schichten erreicht wird. Beispielsweise wird reaktiver Sauerstoff erzeugt, welcher in Wärme reduziert wird, die wiederum zur Hautoberfläche hin abgebaut wird. Die Messbestrahlung findet daher bei dieser Ausführungsform an einer "vorbehandelten" Hautstelle statt, wobei erfindungsgemäß vorzugsweise ein Aufeinandertreffen beider Strahlungsarten gewünscht wird, d.h. die Wärme des Ultraschalls steigt zur Hautoberfläche hin an. Sobald sich diese Wärme in der zu behandelnden Schicht ausbreitet, erfolgt der Lichtimpuls.

Die die entsprechende Hautstelle kennzeichnenden Hautparameter werden daher von einer mit Ultraschall behandelten Hautstelle gewonnen, und die Behandlungsbestrahlung wird auf der Basis dieser Parameter durchgeführt. Hierbei werden die durch den Ultraschall erzeugten positiven Effekte ausgenutzt, beispielsweise eine durch Ultraschall erzeugte Kavitation, welche zu einem Anstieg des Zelldrucks führt, d.h. innerhalb der Materie entsteht ein höherer "mechanischer" Druck, welcher gerade in Bezug auf die Behandlung von Hautunreinheiten gute Erfolge mit sich bringt.

Die Beaufschlagung mit Ultraschall kann auch nur vor der eigentlichen Behandlungsbestrahlung durchgeführt werden.

Eine Beaufschlagung vor der Messbestrahlung und vor der Behandlungsbestrahlung ist ebenfalls möglich.

Die vorliegende Erfindung ist ferner auf eine Vorrichtung zur Durchführung des vorstehend beschriebenen Verfahrens gerichtet. Eine solche Vorrichtung besitzt eine Pulslichtquelle und eine von dieser beaufschlagte Behandlungsbestrahlungseinheit und ist **dadurch gekennzeichnet, dass** sie ferner eine Messbestrahlungseinheit mit Pulslichtquelle und Empfänger oder eine Kamera zur Erstellung eines Hautbildes, eine vom Empfänger oder der Kamera angesteuerte Zentraleinheit zur Auswertung und/oder Verarbeitung der Empfängersignale oder Hautbildsignale und eine Steuereinheit für die Pulslichtquelle der Behandlungsbestrahlungseinheit aufweist, die von der Zentraleinheit angesteuert wird.

Mit der Messbestrahlungseinheit wird die Messbestrahlung des erfindungsgemäßen Verfahrens durchgeführt. Die entsprechende remittierte Strahlung wird vom Empfänger empfangen und in elektrische Signale umgewandelt. Diese Signale werden der Zentraleinheit zugeführt, die die Signale auswertet und/oder verarbeitet. Die Zentraleinheit steuert die Steuereinheit für die Pulslichtquelle der Behandlungsbestrahlungseinheit entsprechend an, wodurch die Bestrahlungsparameter der Pulslichtquelle optimal eingestellt werden.

Anstelle der Messbestrahlungseinheit kann erfindungsgemäß auch eine Kamera zur Erstellung eines Hautbildes Verwendung finden. Hierbei kann die Kamera die Zentraleinheit zur Auswertung und/oder Verarbeitung der Hautbildsignale ansteuern, wobei die Zentraleinheit nach entsprechender Auswertung und/oder Verarbeitung die Steuereinheit für die Pulslichtquelle der Behandlungsbestrahlungseinheit ansteuert. Die Zentraleinheit zerlegt vorzugsweise die empfangenen Hautbilder in einzelne Bildpunkte, deren Hell-Dunkel-Werte bzw. Farbwerte erfasst und mit gespeicherten Standardwerten verglichen werden. Hieraus werden die entsprechenden Behandlungsbestrahlungsparameter ermittelt, die an die Steuereinheit der Behandlungsbestrahlungseinheit weitergegeben werden.

Vorzugsweise besitzen die Behandlungsbestrahlungseinheit und Messbestrahlungseinheit dieselbe Pulslichtquelle. Behandlungsbestrahlungseinheit und Messbestrahlungseinheit können eine Einheit bilden, um die Vorrichtung zu vereinfachen.

Die Behandlungsbestrahlungseinheit und die zugehörige Pulslichtquelle weisen vorzugsweise einen großen Abstand voneinander auf, der über ein lichtleitendes Element überbrückt wird. Dies hat den Vorteil, dass die Haut nicht von der Wärmestrahlung der Pulslichtquelle beeinflusst wird. Eine solche Anordnung kann auch zwischen Messbestrahlungseinheit und der zugehörigen Pulslichtquelle getroffen sein, wenn getrennte Einheiten bzw. getrennte Pulslichtquellen zum Einsatz kommen.

Für die Messbestrahlung und die Behandlungsbestrahlung kann das lichtleitende Element direkt mit der zu enthaarenden Stelle der Haut in Kontakt gebracht werden. Vorzugsweise besitzt die Behandlungsbestrahlungseinheit jedoch einen speziellen Applikator. Einen derartigen Applikator kann auch die Messbestrahlungseinheit aufweisen. Vorzugsweise besitzen beide Einheiten denselben Applikator. Ein solcher Applikator kann optische Bauelemente zur Verformung, Bündelung, Streuung und/oder Spektralmanipulation (Filterung) des Impulslichtes aufweisen.

Wenn ein lichtleitendes Element, insbesondere ein Lichtwellenleiter, Verwendung findet, kann dieses mit einer geeigneten Filtereinrichtung versehen sein.

Es versteht sich, dass auch die verwendete Pulslichtquelle oder die verwendeten Pulslichtquellen optische Bauelemente aufweisen können. Darüber hinaus ist die Pulslichtquelle oder sind die Impulslichtquellen vorzugsweise mit einer Wärmeabsorptionseinrichtung versehen, um die abgestrahlte Wärme zu verringern.

Die Pulslichtquelle umfasst vorzugsweise eine Hochdruckentladungslampe oder entsprechend ausgebildete LED's.

Bei der erfindungsgemäßen Vorrichtung kann auf aufwändige Kühlsysteme verzichtet werden. Die notwendige Wärmeableitung kann durch eine höhere Distanz zwischen Pulslichtquelle und Behandlungsfläche erzielt werden. Zu diesem Zweck wird vorzugsweise die Pulslichtquelle in ein Gehäuse eingebaut und die Strahlung mittels Lichtwellenleiter, welcher während der Behandlung auf die Haut appliziert wird, auf die Haut übertragen. Wie vorstehend erwähnt, kann neben dem direkten Aufsatz eines derartigen Lichtwellenleiters auch ein Applikator (Behandlungskopf) zur Anwendung gelangen. Die von der Pulslichtquelle emittierte Strahlung wird dann beispielsweise über den Lichtwellenleiter auf den Applikator geleitet und durch den Applikator auf die Haut übertragen. Der Applikator kann hierbei insbesondere mit geeigneten Filtereinrichtungen versehen sein. Neben dieser Art der Filterung kann auch eine Filterung innerhalb des lichtleitenden Elementes erfolgen, wie vorstehend erwähnt. Vorzugsweise wird hierbei ein Bragg-Gitter innerhalb des Lichtwellenleiters (Faserbündels) angebracht, welcher durch optische Interferenz die Strahlung entsprechend manipuliert.

Die erfindungsgemäße Vorrichtung kann über mehrere unterschiedliche Applikatoren und mehrere lichtleitende Elemente verfügen.

Als Wärmeabsorptionseinrichtung besitzt die Impulslichtquelle vorzugsweise einen Kupferkern, der die Wärmestrahlung der Impulslichtquelle absorbiert. Der Kupferkern leitet die Wärme über eine Luftkühlung oder Wasserkühlung ab. Auf diese Weise werden am Übertrittspunkt zwischen Pulslichtquelle und lichtleitendem Element (Lichtwellenleiter) thermische Belastungen und daraus resultierende Schädigungen und Degenerationen der Leitungsleistung des lichtleitenden Elementes vermieden.

Des Weiteren kann die Vorrichtung auch innerhalb der Pulslichtquelle über optische Bauelemente (Reflektoren und Transmittoren) verfügen, welche zwischen Pulslichtquelle und lichtleitendem Element angeordnet sind, um die Strahlung zu bündeln, zu streuen, zu verformen oder spektral zu manipulieren (zu filtern). Sowohl die Reflektoren als auch die Filter können in der baulichen Anordnung variabel sein.

Der Applikator ist vorzugsweise so ausgebildet, dass die Größe der Applikationsfläche variiert werden kann. Dies kann beispielsweise über mehrere und verschiedene Frontblenden durchgeführt werden. An der Frontblende des Applikators können ferner Reflektoren angeordnet sein, die die emittierte Strahlung komplett oder teilweise reflektieren. Die Fläche, Beschaffenheit und Formgebung der Reflektoren kann variiert werden, um die Strahlung auf eine größere oder kleinere Behandlungsfläche (Hautoberfläche) zu projizieren.

Bei einer besonders bevorzugten Lösung weist die Behandlungsbestrahlungseinheit bzw. deren Applikator einen Folienfilter auf. Dieser Folienfilter reduziert die Interferenz beim Auftreffen der Behandlungsstrahlung auf die Haut. Er erhöht den Wirkungsgrad der Strahlung und reduziert thermische Effekte auf der Haut. Hierzu besitzt der Folienfilter vorzugsweise eingearbeitete Mikropartikel mit einer Kantenlänge < 30 µm. Dies führt zu einer gleichmäßigen Verteilung der Behandlungsstrahlung, reduziert die Reflektion und Refraktion, so dass eine gleichmäßige Emission in der Wirkebene auftritt.

Der Folienfilter ist vorzugsweise als Gebrauchsfilter ausgebildet. Hierbei wird pro Behandlung ein Filter eingesetzt und nach der Behandlung entsorgt. Dies wiederum reduziert die erforderliche Reinigung der Applikationsfläche, was ansonsten zu einem Verkratzen der Applikationsfläche mit Transmissionsverlusten führen kann. Es versteht sich, dass ein derartiger Folienfilter auch die entsprechende Hygiene verbessert. Der Folienfilter kann sowohl über unterschiedliche Schichtdicken/Materialstärken als auch über zusätzliche Filtereigenschaften im optischen Bereich verfügen.

In Bezug auf die Pulslichtquelle bzw. die Pulslichtquellen können neben üblichen Xenon-Lampen, Xenon-Quecksilberlampen etc. (Hochdruck-Entladungslampen) auch andere emittierende Bauelemente zum Einsatz kommen. Vorzugsweise finden hierbei LED's Anwendung. Dabei werden speziell komplexe Dioden eingesetzt, die über entsprechende optische Bauelemente zur Lichtbündelung verfügen. Diese Dioden können auf eine hohe Lichtausbeute in kurzer Zeit (Blitzlicht) optimiert sein. Solche Dioden können, bedingt durch ihre gegenüber Hochdruckentladungslampen geringere Wärmeentwicklung, sowohl direkt in der Behandlungsbestrahlungseinheit und Messbestrahlungseinheit Verwendung finden als auch in einer externen Pulslichtquelle eingesetzt werden.

Neben üblichen LED's in kompakter Bauform kann auch nur ein Einsatz der emittierenden Chips (Waver) einzeln oder im Verbund erfolgen. Übliche LED-Chips verfügen über eine maximale Kantenlänge von 500 µm. Somit kann durch den Einsatz einzelner Chips im Verbund eine wesentlich höhere Strahlungsstärke bezogen auf die Behandlungsfläche erreicht werden. Diese Chips-Bauweise kann sowohl innerhalb des Applikators oder aber innerhalb der externen Pulslichtquelle verwirklicht werden. Die Anbindung des Lichtwellenleiters erfolgt vorzugsweise faser/chipgebunden, wobei jede Faser des Lichtwellenleiters durch einen separaten Chip versorgt wird.

Vorzugsweise verfügt die Vorrichtung über unterschiedliche und wechselbare LED- und Chip-Einheiten, um notwendige Emissionsspektren zu erzielen. Da die auf LED-Technik basierende Emission teilweise auf nur sehr kurzen Spektralbanden beruht, kann das System auch kombinierte LED-Einheiten mit jeweils unterschiedlichen Spektren (Wellenlängen besitzen. Sowohl die LED- als auch die Chip-Einheiten können über eine Wasser- und/oder Luftkühlung verfügen.

Wenn bei dem erfindungsgemäßen Verfahren eine Beaufschlagung der zu behandelnden Hautstelle mit Ultraschall durchgeführt wird, weist die erfindungsgemäß ausgebildete Vorrichtung eine Einrichtung zur Beaufschlagung der Hautstelle mit Ultraschall auf, die beispielsweise eine Ultraschallstrahlungsquelle, geeignete Ultraschallleiteinrichtungen sowie geeignete Hautapplikationseinrichtungen umfasst.

Die Zentraleinheit der erfindungsgemäßen Vorrichtung kann als integrierte Rechnereinheit ausgebildet sein. Diese Rechnereinheit kann entsprechende Eingabe/Ausgabeeinheiten aufweisen, beispielsweise über zusätzliche Tasten als auch über Touchscreen-Display-Oberflächen bedient werden. Ferner kann die Rechnereinheit über eine Chipkarten-Leseeinheit verfügen. Schließlich kann die Einheit entsprechende Schnittstellen aufweisen, um ein Anschließen an andere Geräte zu ermöglichen.

Die Erfindung wird nachfolgend anhand eines Ausführungsbeispiels in Verbindung mit der Zeichnung im Einzelnen erläutert. Es zeigen:
- Figur 1: den schematischen Aufbau eines Ausführungsbeispiels einer Vorrichtung zur Haarentfernung; und
- Figur 2: eine Darstellung des Applikators und der Pulslichtquelle der Figur 1.

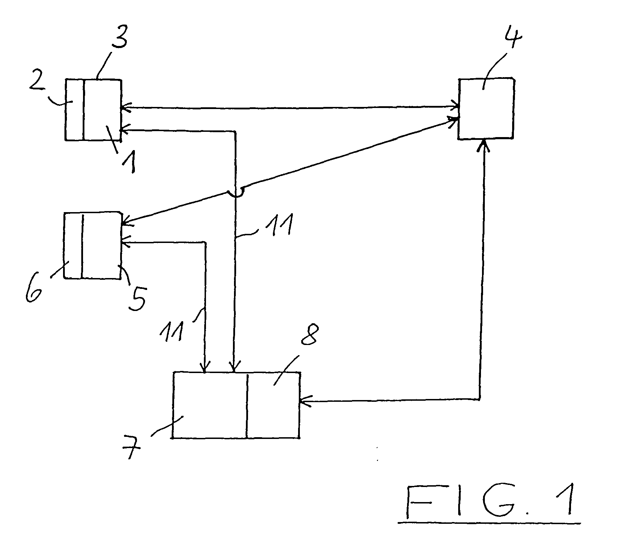

Die in Figur 1 schematisch dargestellte Vorrichtung zur Haarentfernung besitzt eine Messbestrahlungseinheit mit einem Applikator 2 und einem Empfänger 3, eine Behandlungsbestrahlungseinheit 5 mit einem Applikator 6, eine Zentraleinheit 4 und eine Pulslichtquelle 7 mit zugehöriger Steuereinheit 8. In dieser Figur sind die Messbestrahlungseinheit 1 und die Behandlungsbestrahlungseinheit 5 als getrennte Einheiten dargestellt. In der Praxis sind jedoch normalerweise beide Einheiten zu einer Einheit zusammengefasst.

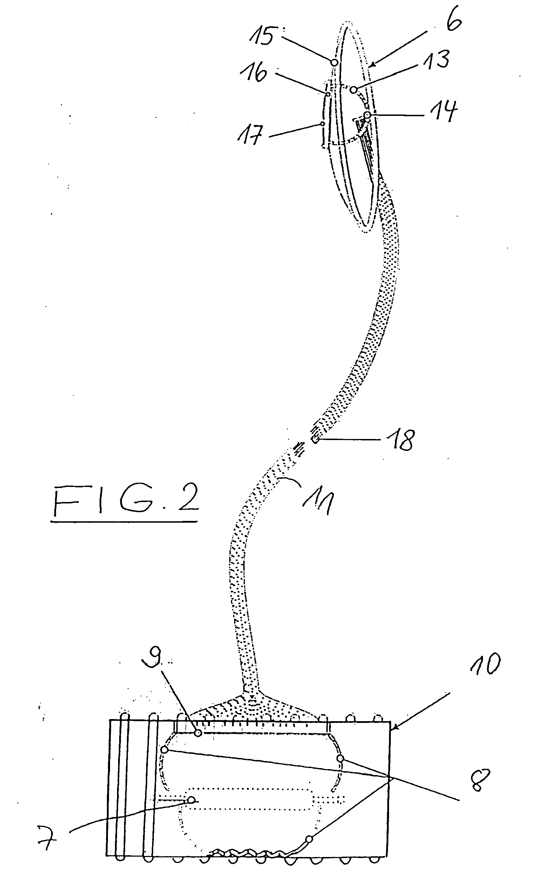

Die Pulslichtquelle 7 beaufschlagt sowohl die Messbestrahlungseinheit 1 als auch die Behandlungsbestrahlungseinheit 5 mit Pulslicht über geeignete Lichtwellenleiter 11. Zu Beginn des Verfahrens zur Haarentfernung wird die Messbestrahlungseinheit 1 mit Pulslicht beaufschlagt. Die von der entsprechenden Haut remittierte Strahlung wird vom Empfänger 3 der Messbestrahlungseinheit empfangen und in elektrische Impulse umgewandelt, die der Zentraleinheit 4 zur Auswertung bzw. Verarbeitung zugeführt werden. Die Zentraleinheit 4 ermittelt aus den empfangenen Werten, die die beaufschlagte Hautstelle charakterisieren, entsprechende Steuerparameter für die Pulslichtquelle und sendet diese an die Steuereinheit 8 für die Pulslichtquelle. In der nun folgenden Behandlungsbestrahlung beaufschlagt die Pulslichtquelle 7 über den Lichtwellenleiter 11 die Behandlungsbestrahlungseinheit mit für die jeweilige Hautstelle optimiertem Pulslicht. Die Beaufschlagung der Hautstelle mit Pulslicht erfolgt dabei über den Applikator 6, der in Verbindung mit Figur 2 im Einzelnen erläutert wird. Die Zentraleinheit 4 steuert sowohl die Funktion der Messbestrahlungseinheit 1 und Behandlungsbestrahlungseinheit 5 als auch die Funktion der Pulslichtquelle 7.

Figur 2 zeigt den schematischen Aufbau der Behandlungsbestrahlungseinheit 5, mit Lichtwellenleiter 11 und Pulslichtquelle 7. Die Pulslichtquelle 7 besitzt eine geeignete Emissionsquelle (Lampe) die zur Abgabe des gewünschten Pulslichtes von der Steuereinheit 8 entsprechend angesteuert wird. Als zusätzliche optische Bauteile weist die Pulslichtquelle 7 einen Reflektor (Transmittor) und einen Filter 9 auf. Die gesamte Einheit ist in einem Kupferkern untergebracht. Das von der Emissionsquelle abgegebene Pulslicht wird über einen Lichtwellenleiter 11 mit entsprechenden Lichtwellenleiterfasern 18 der Behandlungsbestrahlungseinheit zugeführt, die den dargestellten Applikator 6 mit einer entsprechenden Applikationsfläche 16 aufweist. Auf der Applikationsfläche befindet sich ein austauschbarer Folienfilter 17. Ferner besitzt der Applikator 6 eine geeignete Frontblende 15, einen Reflektor 13 sowie einen Filter 14.

Im Behandlungsbestrahlungsbetrieb wird Pulslicht über den Lichtwellenleiter 11 dem Applikator 6 zugeführt, der das Pulslicht über den Folienfilter 17 und die Applikationsfläche 16 auf die entsprechende Hautstelle aufbringt. Die Behandlung wird vorzugsweise kontinuierlich durchgeführt, so dass der Applikator kontinuierlich von einem zum anderen zu enthaarenden Hautbereich bewegt wird.

## Patentansprüche

1. Verfahren zur kosmetischen Hautbehandlung, insbesondere kosmetischen Haarentfernung, mit den folgenden Schritten:
a. Durchführen einer Messbestrahlung vor einer Behandlungsbestrahlung durch Beaufschlagen der zu behandelnden, insbesondere zu enthaarenden, Hautstelle mit Pulslicht oder Aufnahme der zu behandelnden, insbesondere zu enthaarenden, Hautstelle mit einer Kamera vor einer Behandlungsbestrahlung;
b. Empfangen und Auswerten der remittierten Messstrahlung oder Erstellen eines Hautbildes mit der Kamera und Auswerten desselben zur Bestimmung von den akuten Hautzustand der zu behandelnden, insbesondere zu enthaarenden, Hautstelle kennzeichnenden Hautparametern;
c. Einstellen von Parametern der Behandlungsbestrahlung in Abhängigkeit von den bestimmten Hautparametern; und
d. Durchführen der Behandlungsbestrahlung mit den eingestellten Behandlungsbestrahlungsparametern durch Beaufschlagen der zu behandelnden, insbesondere zu enthaarenden, Hautstelle mit Pulslicht.

2. Verfahren nach Anspruch 1, **dadurch gekennzeichnet, dass** es kontinuierlich durchgeführt wird.

3. Verfahren nach Anspruch 2, **dadurch gekennzeichnet, dass** abwechselnd eine Messbestrahlung oder eine Hautbilderstellung und eine nachfolgende Behandlungsbestrahlung durchgeführt werden.

4. Verfahren nach Anspruch 3, **dadurch gekennzeichnet, dass** von der Durchführung einer Messbestrahlung oder Hautbilderstellung und einer nachfolgenden Behandlungsbestrahlung einer zu behandelnden, insbesondere zu enthaarenden, Hautstelle kontinuierlich auf die Durchführung einer Messbestrahlung oder Hautbilderstellung und einer nachfolgenden Behandlungsbestrahlung einer benachbarten zu behandelnden, insbesondere zu enthaarenden, Hautstelle übergegangen wird.

5. Verfahren nach einem der vorangehenden Ansprüche, **dadurch gekennzeichnet, dass** mit Pulslicht einer Wellenlänge von 400 nm bis 1.200 nm, insbesondere 620 nm bis 1.200 nm, einer Puls- und Pausenlänge von je 2-50 msec und/oder einer Pulsintensität von 1-70 J/cm² gearbeitet wird.

6. Verfahren nach einem der vorangehenden Ansprüche, **dadurch gekennzeichnet, dass** aus den Absorptions-, Refraktions- und/oder Reflektionsparametern der remittierten Messstrahlung oder den Hell-Dunkel-Werten bzw. Farbwerten des erstellten Hautbildes die den akuten Hautzustand kennzeichnenden Hautparameter, insbesondere die Dicke der Hautschichten und/oder der Melanosomtyp, bestimmt werden.

7. Verfahren nach einem der vorangehenden Ansprüche, **dadurch gekennzeichnet, dass** als Parameter der Behandlungsbestrahlung die Wellenlänge, die Puls/Pausenlänge und/oder die Pulsintensität eingestellt werden.

8. Verfahren nach einem der vorangehenden Ansprüche, **dadurch gekennzeichnet, dass** durch Temperaturmessung der Haarpapille ein die Vorbelastung der Haut mit Strahlung kennzeichnender Parameter bestimmt wird.

9. Verfahren nach einem der vorangehenden Ansprüche, **dadurch gekennzeichnet, dass** eine Behandlungsbestrahlung mit einem geänderten Anwendungsspektrum, insbesondere einer Anreicherung der grünen und blauen Spektralbande, eingesetzt wird.

10. Verfahren nach einem der vorangehenden Ansprüche, **dadurch gekennzeichnet, dass** vor der Durchführung der Messbestrahlung und/oder Behandlungsbestrahlung die zu behandelnde Hautstelle mit Ultraschall beaufschlagt wird.

11. Vorrichtung zur Durchführung des Verfahrens nach einem der vorangehenden Ansprüche mit einer Pulslichtquelle und einer von dieser beaufschlagten Behandlungsbestrahlungseinheit, **dadurch gekennzeichnet, dass** sie ferner eine Messbestrahlungseinheit (1) mit Pulslichtquelle (7) und Empfänger (3) oder eine Kamera zur Erstellung eines Hautbildes, eine vom Empfänger (3) oder der Kamera angesteuerte Zentraleinheit (4) zur Auswertung und/oder Verarbeitung der Empfängersignale oder Hautbildsignale und eine Steuereinheit (8) für die Pulslichtquelle (7) der Behandlungsbestrahlungseinheit (5) aufweist, wobei die Steuereinheit (8) von der Zentraleinheit (4) angesteuert wird.

12. Vorrichtung nach Anspruch 11, **dadurch gekennzeichnet, dass** die Behandlungsbestrahlungseinheit (5) und Messbestrahlungseinheit (1) dieselbe Pulslichtquelle (7) besitzen.

13. Vorrichtung nach Anspruch 11 oder 12, **dadurch gekennzeichnet, dass** Behandlungsbestrahlungseinheit (5) und Messbestrahlungseinheit (1) eine Einheit bilden.

14. Vorrichtung nach einem der Ansprüche 11 bis 13, **dadurch gekennzeichnet, dass** die Behandlungsbestrahlungseinheit (5) und die zugehörige Pulslichtquelle (7) einen großen Abstand voneinander aufweisen, der über ein lichtleitendes Element (11) überbrückt wird.

15. Vorrichtung nach einem der Ansprüche 11 bis 14, **dadurch gekennzeichnet, dass** die Behandlungsbestrahlungseinheit (5) einen Applikator (6) besitzt.

16. Vorrichtung nach Anspruch 15, **dadurch gekennzeichnet, dass** der Applikator (6) optische Bauelemente zur Verformung, Bündelung, Streuung und/oder Spektralmanipulation (Filterung) des Impulslichtes aufweist.
